# EUROPÄISCHE PATENTSCHRIFT

(11) **EP 2 578 171 B1**
(45) Veröffentlichungstag und Bekanntmachung des Hinweises auf die Patenterteilung: **06.05.2015**
(21) Anmeldenummer: 12198512.1
(22) Anmeldetag: 02.03.2007
(51) Int. Cl.: A61B 17/32, A61B 17/16, A61B 17/28, A61B 19/00, A61B 10/06

(54) **Chirurgisches Instrument**
Surgical instrument
Instrument chirurgical

(30) Priorität: 17.03.2006 DE 102006012754
(43) Veröffentlichungstag der Anmeldung: 10.04.2013
(62) Teilanmeldung aus: 07703575.6
(73) Patentinhaber: Karl Storz GmbH & Co. KG, 78532 Tuttlingen (DE)
(72) Erfinder: Widmann, Heinrich, 88637 Buchheim (DE)
(74) Vertreter: Daub, Thomas

(56) Entgegenhaltungen:
- DE-A1- 4 316 768
- DE-A1- 19 513 572
- US-A- 5 961 531

## Beschreibung

Die Erfindung betrifft ein chirurgisches Instrument mit einem Schaft und einem an dem Schaft gleitbar gelagerten Schieber, wobei zumindest ein in zumindest einer Längsnut geführtes Führungselement an Schaft bzw. Schieber vorgesehen ist und Schaft bzw. Schieber jeweils mit einem Griffteil verbunden sind, von denen ein Griffteil gegenüber dem anderen Griffteil um ein Drehgelenk herum bewegbar und mit einem Schlitz versehen ist, in den ein Querriegel des Schiebers eingreift.

### Stand der Technik

Derartige Instrumente werden als Schiebeschaftinstrumente bezeichnet. Mit ihnen werden schneidende, scherende, klemmende oder dgl. Eingriffe, beispielsweise im menschlichen Körper, vorgenommen. Hierbei wird über Betätigung der Griffteile der Schieber auf einer Gleitfläche des Schafts bewegt und an dessen Ende ein Arbeitselement betätigt.

Vor allem finden derartige Schiebeschaftinstrumente als Knochenstanzen, Ohrzangen und in der gynäkologischen Biopsie Anwendung. Dabei ist ein generelles Problem, dass es sich bei derartigen chirurgischen Instrumenten um Produkte mit verschiedensten Gelenken, Führungen oder Nuten und Rinnen handelt. Derartige Instrumente sind deshalb außerordentlich schwer zu reinigen und zu sterilisieren, wobei aber gerade die Reinigung chirurgischer Instrumente äußerst wichtig ist. Die Hygieneanforderungen für solche chirurgischen Instrumente steigen durch die Gefahr der Übertragung von Krankheiten, beispielsweise von Hepatitis und Aids, stark an.

Ein Instrument der o.g. Art ist beispielsweise aus der DE-U-20103630 und DE-A-19513572 bekannt.

### Aufgabe

Aufgabe der vorliegenden Erfindung ist es, ein Instrument der o.g. Art weiter zu verbessern und zu vereinfachen.

### Lösung der Aufgabe

Zur Lösung der Aufgabe führt, dass die Bewegung des Schiebers an dem Schaft mit einer Verriegelungseinrichtung begrenzbar ist. Wenn diese Verriegelung aufgehoben wird, sollen Schieber und Schaft um ein Drehgelenk scherenartig voneinander trennbar sein.

In einem Ausführungsbeispiel ist vorgesehen, dass sich das Drehgelenk zwischen dem Schaft und einem feststehenden Griffteil befindet, bei einem zweiten Ausführungsbeispiel befindet sich das Drehgelenk zwischen dem Schieber und einem bewegbaren Griffteil.

Des Weiteren ist ein erfindungsgemäßer Gedanke, dass dem Schieber noch ein Schubteil zugeordnet ist, wobei das Schubteil mit dem bewegbaren Griffteil einerseits und andererseits über das Drehgelenk mit dem Schieber gekoppelt ist. Dabei ist daran gedacht, dass das Schubteil mit dem feststehenden Griffteil verbunden bleibt, während der Schieber durch Betätigen der Verriegelungseinrichtung freigegeben wird. In einem bevorzugten Ausführungsbeispiel soll es jedoch auch möglich sein, nach Betätigen der Verriegelungseinrichtung durch eine weitere Bewegung des Schiebers oder Schubteils diesen von dem Schaft freizugeben, wobei jeweils die Führungselemente aus den entsprechenden Längsnuten führbar sind.

Je nach Ausführungsbeispiel können sich die Führungselemente bzw. Längsnuten am oder im Schieber befinden. Auch bezüglich der Verriegelungseinrichtungen sind eine Reihe von Möglichkeiten denkbar, die in den Unteransprüchen beschrieben sind. Besonders bevorzugt wird eine Verriegelungseinrichtung, welche das bewegbare Griffteil hintergreift und als Kipphebel in einer entsprechenden Ausnehmung in dem feststehenden Griffteil bewegbar ist. Wird der Anschlag bzw. eine Anschlagkante des Kipphebels, auf welche das bewegbare Griffteil auftrifft, abgestuft ausgebildet, so kann die Öffnungsweite der beiden Griffteile zueinander variiert werden, was die Möglichkeit der Trennung von Schieber und Schaft erheblich variiert.

Es wird ein chirurgisches Instrument nach wenigstens einem der Ansprüche 7 bis 11 vorgeschlagen, wobei der Anschlag mit einem auf dem Schieber sitzenden Kipphebel verbunden ist.

Ferner wird ein chirurgisches Instrument nach wenigstens einem der Ansprüche 7 bis 11 vorgeschlagen, wobei der Anschlag mit einem auf dem Schieber sitzenden Drehhebel verbunden ist.

Ferner wird ein chirurgisches Instrument nach wenigstens einem der Ansprüche 1 bis 15 und/oder nach einem der beiden vorherigen Absätze vorgeschlagen, wobei die Bewegung des beweglichen Griffteils durch einen Anschlag begrenzt ist.

Ferner wird ein chirurgisches Instrument nach dem vorherigen Absatz vorgeschlagen, wobei der Anschlag ein Kipphebel ist.

Ferner wird ein chirurgisches Instrument nach den beiden vorherigen Absätzen vorgeschlagen, wobei der Anschlag zumindest zwei, bevorzugt drei Öffnungsstellungen des beweglichen Griffteils zulässt.

Ferner wird ein chirurgisches Instrument nach einem der drei vorherigen Absätze vorgeschlagen, wobei der Anschlag im feststehenden Griffteil hinter dem beweglichen Griffteil sitzt und um eine Achse schwenkbar ist.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine Draufsicht auf ein erfindungsgemäßes chirurgisches Instrument;
Figur 2 eine Draufsicht auf das chirurgische Instrument gemäß Figur 1 in einer weiteren Gebrauchslage;
Figur 3 einen vergrößert dargestellten geschnittenen Teilbereich aus dem chirurgischen Instrument gemäß Figur 2;
Figur 4 eine Draufsicht auf ein weiteres Ausführungsbeispiel eines chirurgischen Instruments in Explosionsdarstellung;
Figur 5 eine Draufsicht auf ein weiteres Ausführungsbeispiel eines chirurgischen Instruments;
Figur 6 eine Draufsicht auf das chirurgische Instrument gemäß Figur 5 in einer weiteren Gebrauchslage;
Figur 7 eine Draufsicht auf ein weiteres Ausführungsbeispiel eines chirurgischen Instruments;
Figur 8 eine vergrößert dargestellte Unteransicht eines Teilbereichs eines Schiebers aus Figur 7;
Figur 9 eine vergrößerte dargestellte Unteransicht eines Teilbereichs des Schiebers gemäß Figur 7 in einer weiteren Gebrauchslage;
Figur 10 eine Draufsicht auf das chirurgische Instrument gemäß Figur 7 in einer weiteren Gebrauchslage;
Figur 11 eine Draufsicht auf ein weiteres Ausführungsbeispiel eines chirurgischen Instruments;
Figur 12 eine Draufsicht auf ein weiteres Ausführungsbeispiel eines chirurgischen Instruments;
Figur 13 eine Draufsicht auf das chirurgische Instrument gemäß Figur 12 in einer weiteren Gebrauchslage;
Figur 14 eine Draufsicht auf ein weiteres Ausführungsbeispiel eines chirurgischen Instruments;
Figuren 15 und 16 vergrößert dargestellte perspektivische Ansichten eines Ausschnitts aus Figur 14 in unterschiedlichen Stellungen eines Kipphebels;
Figur 17 eine Draufsicht auf das chirurgische Instrument gemäß Figur 14 in einer weiteren Gebrauchslage;
Figur 18 eine Draufsicht auf ein nicht erfindungsgemäßes chirurgisches Instrument beim Zusammenbau zu einer Veranschaulichung der Erfindung;
Figuren 19 und 20 zwei Draufsichten auf das nicht erfindungsgemäße chirurgische Instrument gemäß Figur 18 in unterschiedlichen Gebrauchslagen;
Figur 21 eine Draufsicht auf einen vergrößert dargestellten Druckkopf eines nicht erfindungsgemäßen chirurgischen Instruments gemäß Figur 18 mit abragendem gestuftem Stift.

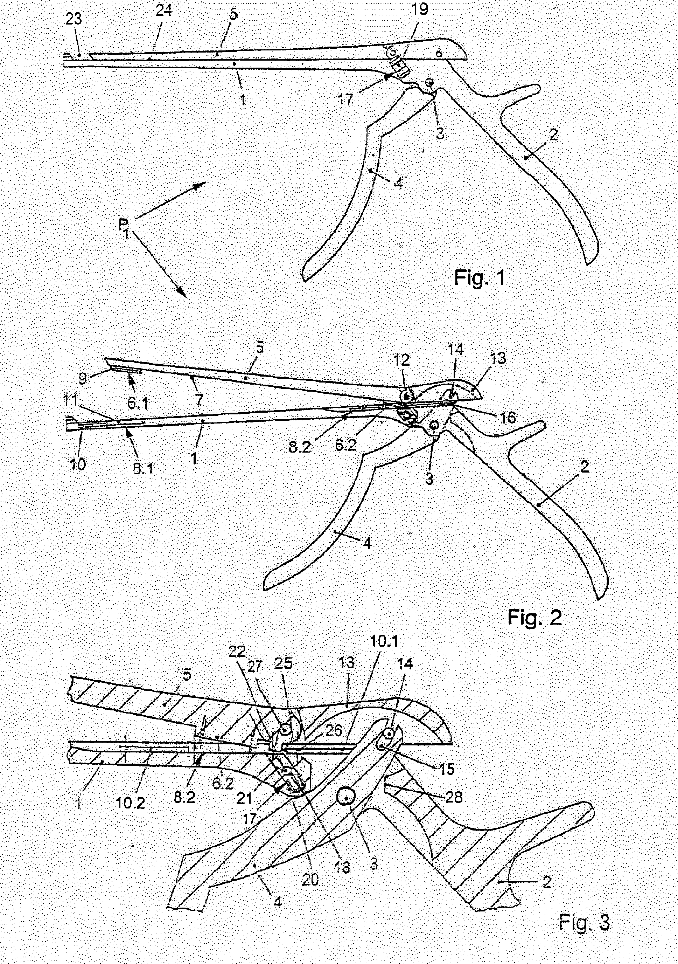

Ein erfindungsgemäßes chirurgisches Instrument P₁ weist gemäß den Figuren 1 und 2 einen Schaft 1 auf, der mit einem feststehenden Griffteil 2 verbunden ist. Dieses feststehende Griffteil 2 ist ferner über ein Drehgelenk 3 mit einem bewegbaren Griffteil 4 verbunden, wobei dieses bewegbare Griffteil 4 um das Drehgelenk 3 geschwenkt werden kann. Durch die Bewegung des bewegbaren Griffteils 4 wird ein Schieber 5 entlang des Schafts 1 bewegt. Dabei greifen Führungselement 6.1 und 6.2, die an einer Unterfläche 7 des Schiebers 5 vorgesehen sind, in Längsnuten 8.1 und 8.2 in dem Schaft 1 ein, wobei die Führungselemente 6.1 und 6.2 umgedreht P-förmig ausgestaltet sind, so dass seitliche Schienen 9 an den Führungselementen 6.1 bzw. 6.2 in hinterschnittenen Nuten 10 und 10.2 in den Längsnuten 8.1 und 8.2 geführt werden können. Teilbereiche 11 dieser Längsnuten 8.1 und 8.2 sind jedoch mit kleinen hinterschnittenen Nuten 10 und 10.2 versehen, so dass aus diesen Teilbereichen 11 das Führungselement 6.1 bzw. 6.2 herausgehoben werden kann. In diesem Fall machen Schaft 1 und Schieber 5 zueinander eine zangenartige Bewegung, wobei der Schieber 5 um ein Drehgelenk 12 dreht. Über dieses Drehgelenk 12 ist der Schieber 5 mit einem Schubteil 13 verbunden, von dem unterhalb eines Querriegels 14, der in einen Schlitz 15 (siehe Figur 3) in dem bewegbaren Griffteil 4 eingreift, mit Führungselementen 16 versehen ist, die wiederum in hinterschnittenen Nuten 10.1 in Fortsetzung der Längsnut 8.2 geführt sind.

Zur Begrenzung der Bewegung des Schiebers 5 gegenüber dem Schaft 1 ist eine Verriegelungseinrichtung 17 vorgesehen, wie sie in der DE 201 03 630 A1 beschrieben ist. In dieser Verriegelungseinrichtung 17 befindet sich ein Bolzen als Anschlag 18, der durch Betätigung mittels eines Schieberknopfes 19 gegen die Kraft einer nicht näher gezeigten Schraubenfeder, welche sich in einem Raum 20 eines Sacklochs 21 befindet, aus der lichten Weite der Längsnut 8.2 geschoben werden kann. Zwischen dem Schieberknopf 19 und dem Anschlag 18 befindet sich ein Verbindungsstift 22.

Die Funktionsweise der vorliegenden Erfindung ist folgende:

In Gebrauchslage befinden sich die Führungselemente 6.1, 6.2 und 16 in Eingriff mit den entsprechenden hinterschnittenen Nuten 10, 10.1 und 10.2, so dass der Schieber 5 bei Betätigung des bewegbaren Griffteils 4 entlang dem Schaft 1 verschoben werden kann, wobei mittels beispielsweise einem Stanzmaul 23 eine Gewebeprobe entnommen wird.

Soll das Instrument beispielsweise gereinigt werden, so gilt dies vor allem für den Zwischenraum 24 zwischen dem Schaft 1 und dem Schieber 5, da sich in diesem Zwischenraum 24 Blut bzw. Gewebeflüssigkeit ansammeln kann. Zur Erleichterung dieser Reinigung ist es ratsam, den Schieber 5 von dem Schaft 1 zu trennen. Dies geschieht dadurch, dass die Verriegelungseinrichtung 17 betätigt wird, d.h., der Anschlag 18 wird durch Betätigung des Schieberknopfes 19 in dem Sackloch 21 nach unten verschoben, so dass der Anschlag 18 aus der lichten Weite der Längsnut 8.2 herausgefahren wird. Hierdurch können der Schieber 5 und auch das Schubteil 13 weiter nach rechts verfahren werden, so dass die Führungselemente 6.1 und 6.2 in einen Bereich der Längsnuten 8.1 und 8.2 gelangen, in denen sie nicht mehr in hinterschnittenen Nuten 10 bzw. 10.2 gefangen sind. Dabei trifft eine hintere Kante 25 des Führungselements 6.2 auf eine Steuerkante 26 in der Längsnut 8.2, so dass der Schieber 5 um das Drehgelenk 12 und hier insbesondere um einen Querbolzen 27 gedreht wird. Hierdurch wird der Schieber 5 von dem Schaft 1 abgehoben. Dieses Öffnen genügt in der Regel für eine ausreichende Reinigungsstellung des Instruments.

Sollte jedoch der Schieber 5 gänzlich von dem Schaft 1 entfernt werden, so kann das bewegbare Griffteil 4 noch weiter um das Drehgelenk 3 geöffnet werden, so dass letztendlich auch die Führungselemente 16 des Schubteils 13 außer Eingriff mit den hinterschnittenen Nuten 10.1 gelangen. Damit kann auch das Schubteil 13 von dem feststehenden Griffteil 2 abgenommen werden, wobei der Querriegel 14 aus dem Schlitz 15 gleitet.

Soll das Abnehmen des Schiebers 5 bzw. Schubteils 13 von dem Schaft 1 bzw. dem feststehenden Griffteil 2 verhindert werden, so kann in dem feststehenden Griffteil 2 nahe dem bewegbaren Griffteil 4 ein Anschlag 28 vorgesehen werden, der gewährleistet, dass das bewegbare Griffteil 4 nicht soweit geöffnet wird, dass die Führungselemente 16 außer Eingriff mit den hinterschnittenen Nuten 10.1 gelangen.

Beim Zusammenbau genügt es, den Schieber 5 und den Schaft 1 zusammenzudrücken, so dass das Führungselement 6.2 den Anschlag 18 in dem Sackloch 21 nach unten drückt. Wird jetzt das bewegbare Griffteil 4 betätigt, wird der Schieber 5 über das Schubteil 13 nach vorne verschoben, wobei das Führungselement 6.2 den Anschlag 18 überfährt, der dann hinter das Führungselement 6.2 unter dem Druck der Schraubenfeder in die lichte Weite der Längsnut 8.2 einfährt. In diesem Augenblick sind aber die Führungselemente 6.1 und 6.2 bereits von den hinterschnittenen Nuten 10 und 10.2 gefangen, so dass kein Abheben des Schiebers 5 vom Schaft 1 erfolgen kann.

Sollte das Schubteil 13 zusammen mit dem Schieber 5 von dem Schaft 1 bzw. dem feststehenden Griffteil 2 abgenommen worden sein, so müssen selbstverständlich vor dem Zusammenpressen von Schieber 5 und Schaft 1 die Führungselemente 16 in die hinterschnittenen Nuten 10.1 eingesetzt werden.

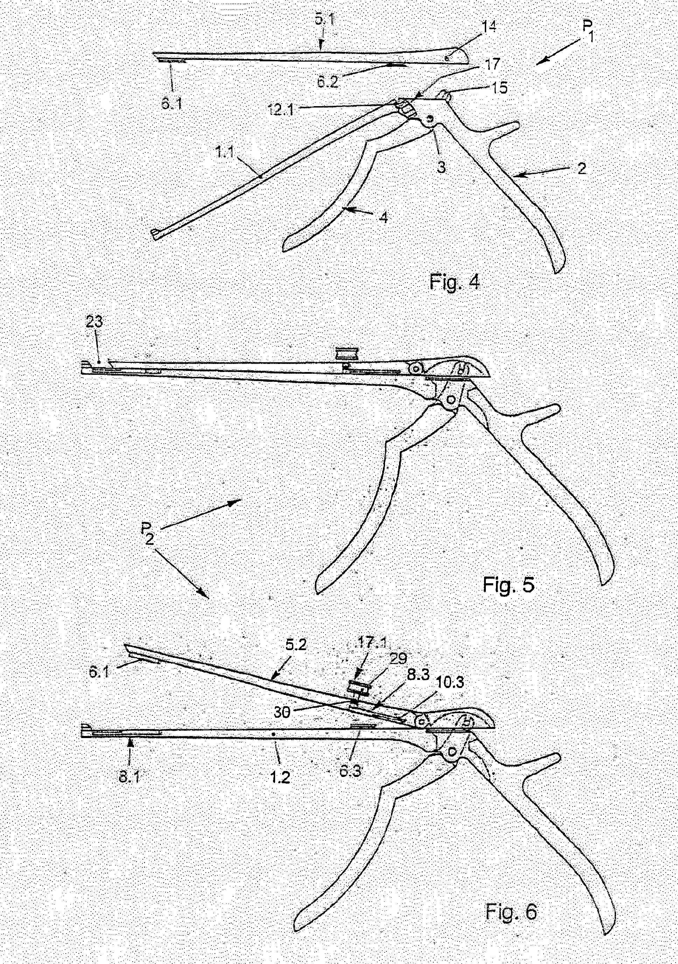

In Figur 4 ist ein weiteres Ausführungsbeispiel eines chirurgischen Instruments P₁ gezeigt. Das feststehende Griffteil 2 und das bewegbare Griffteil 4 entsprechen denjenigen nach den Figuren 1 und 2, ebenso das Drehgelenk 3, die Verriegelungseinrichtung 17, der Querriegel 14 und der Schlitz 15. An dem Schieber 5.1, der in diesem Fall kein Drehgelenk aufweist, sondern bei dem das Schubteil Teil des gesamten Schiebers 5.1 ist, sind ebenfalls die Führungselemente 6.1 und 6.2 vorgesehen.

Unterschiedlich ist jedoch, dass der Schaft 1.1 nicht einstückig mit dem feststehenden Griffteil 2 verbunden, sondern über ein Drehgelenk 12.1 an dieses angelenkt ist. Ferner fehlt das hintere Führungselement 16, so dass nach Betätigen der Verriegelungseinrichtung 17 und Abklappen des Schafts 1.1 der Schieber 5.1 gänzlich von dem Schaft 1.1 und dem feststehenden Griffteil 2 freikommt.

Das Ausführungsbeispiel eines chirurgischen Instruments P₂ gemäß den Figuren 5 und 6 unterscheidet sich von den oben beschriebenen Ausführungsbeispielen durch die Ausgestaltung der Verriegelungseinrichtung und die Anordnung der Führungselemente und Längsnuten. Während im vorderen Bereich nahe dem Stanzmaul 23 nach wie vor das Führungselement 6.1 von dem Schieber 5.2 abragt und in die Längsnut 8.1 in dem Schaft 1.2 eingreift, ragt im proximalen Bereich ein Führungselement 6.3 von dem Schaft 1.2 auf und wirkt mit einer Längsnut 8.3 in dem Schieber 5.2 zusammen. Die entsprechenden hinterschnittenen Nuten 10.3 in der Längsnut 8.3 sind in diesem Fall auch hinter dem Führungselement 6.3 vorgesehen. Dagegen befindet sich die Verriegelungseinrichtung 17.1 vor dem Führungselement 6.3. Dieses Verriegelungselement 17.1 besitzt einen Zugknopf 29, mit dem ein als Stift 30 ausgebildeter Anschlag aus der lichten Weite der Längsnut 8.3 gezogen werden kann. Damit kann der Schieber 5.2 weiter nach links verschoben werden, so dass das Führungselement 6.3 in einen Bereich der Längsnut 8.3 gelangt, der nicht mehr von der hinterschnittenen Nut 10.3 belegt ist. Hierdurch gibt das Führungselement 6.3 den Schieber 5.2 frei, so dass dieser aufklappen kann, da auch gleichzeitig das Führungselement 6.1 in der Längsnut 8.1 freigegeben ist.

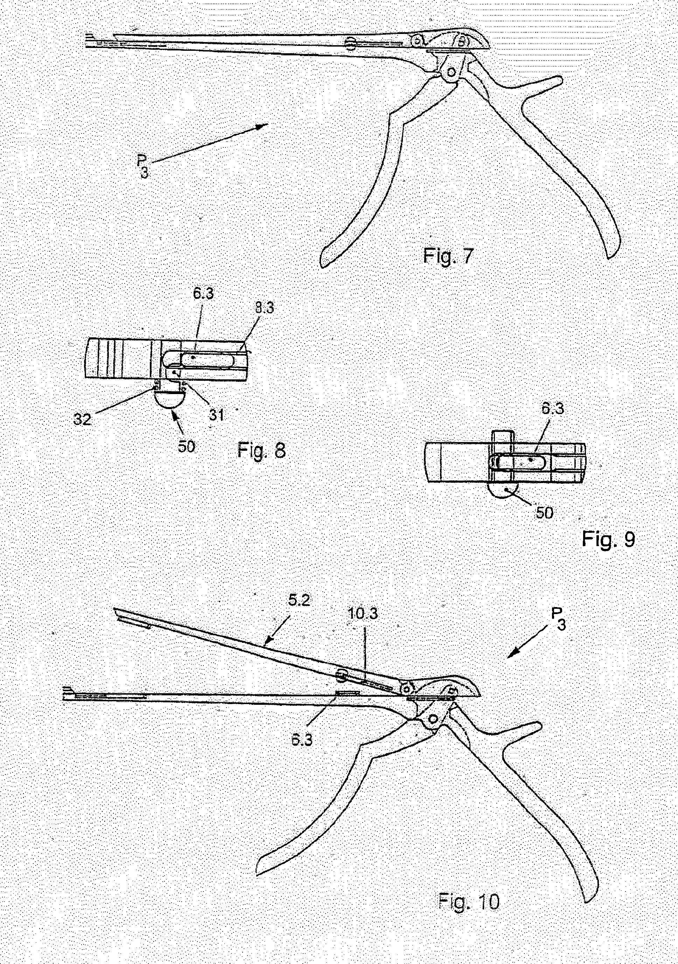

Das Ausführungsbeispiel eines erfindungsgemäßen chirurgischen Instruments P₃ gemäß den Figuren 7 bis 10 unterscheidet sich von den oben beschriebenen ebenfalls durch die Ausgestaltung der Verriegelungseinrichtung. Es ähnelt im Wesentlichen dem Ausführungsbeispiel gemäß Figur 5 und 6, jedoch befindet sich seitlich an dem Schieber 5.2 ein als Druckknopf 50 ausgebildeter Anschlag, in dem eine Ausnehmung 31 eingeformt ist. Wird der Druckknopf 50 gegen die Kraft einer Feder 32 gedrückt, so gelangt die Ausnehmung 31 in Übereinstimmung mit der Längsnut 8.3, so dass das Führungselement 6.3, wie in Figur 9 gezeigt, in die Ausnehmung 31 einfahren kann, so dass es aus den hinterschnittenen Nuten 10.3 gleitet und der Schieber 5.2 freigegeben wird.

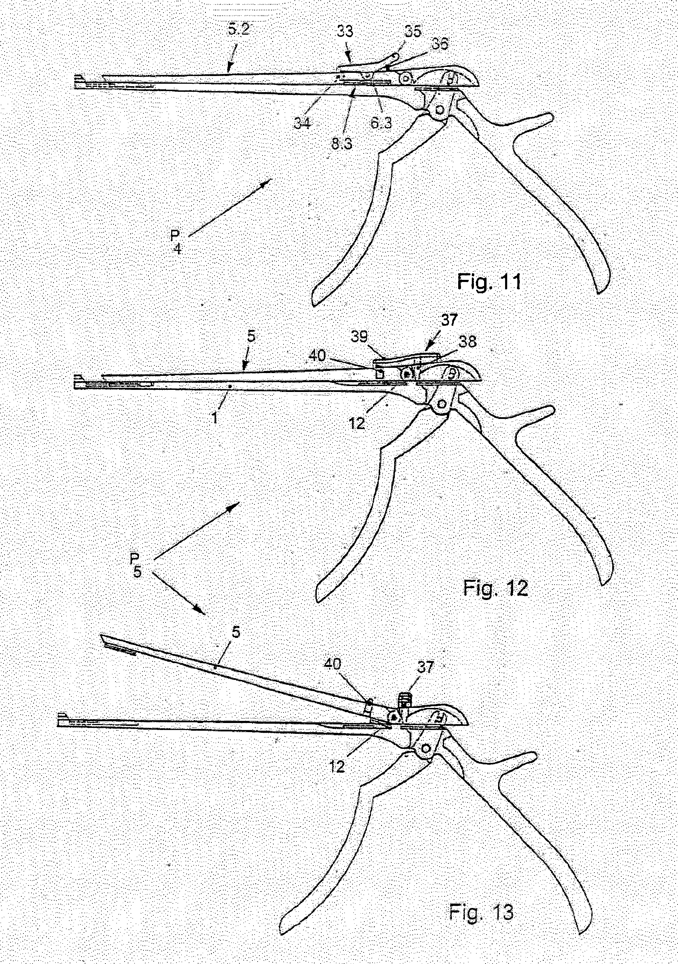

Bei dem Ausführungsbeispiel eines chirurgischen Instruments P₄ gemäß Figur 11 sitzt auf dem Schieber 5.2 ein Kipphebel 33 auf, von dem eine Nase 34 als Anschlag abragt, die vor dem Führungselement 6.3 in die lichte Weite der Längsnut 8.3 eingreift. Wird der Kipphebel 33 durch Druck auf den Hebelarm 35 gegen die Kraft einer Feder 36 betätigt, so wird die Nase 34 aus der lichten Weite der Längsnut 8.3 entfernt, so dass das Führungselement 6.3 einen Bereich in der Längsnut 8.3 erreichen kann, in welchem es freigegeben ist.

Bei dem Ausführungsbeispiel eines chirurgischen Instruments P₅ gemäß den Figuren 12 und 13 befindet sich auf dem Schieber 5 ein Drehhebel 37. Der Drehhebel 37 ist um eine Drehachse 38 drehbar, wobei sich die Drehachse 38 hinter dem Drehgelenk 12 befindet. In der in Figur 12 gezeigten Gebrauchslage übergreift der Drehhebel 37 mit einem vorderen Druckarm 39 den Schieber 5, so dass der Schieber 5 in Anlage an den Schaft 1 gehalten ist. Diese Lage wird durch einen federgelagerten Raststift 40 in dem Schieber 5 gesichert.

Wird der Drehhebel 37, wie in Figur 13 gezeigt, um 90° gedreht, wird auch der Druckarm 39 von dem Schieber 5 entfernt, so dass der Schieber 5 um das Drehgelenk 12 aufklappen kann.

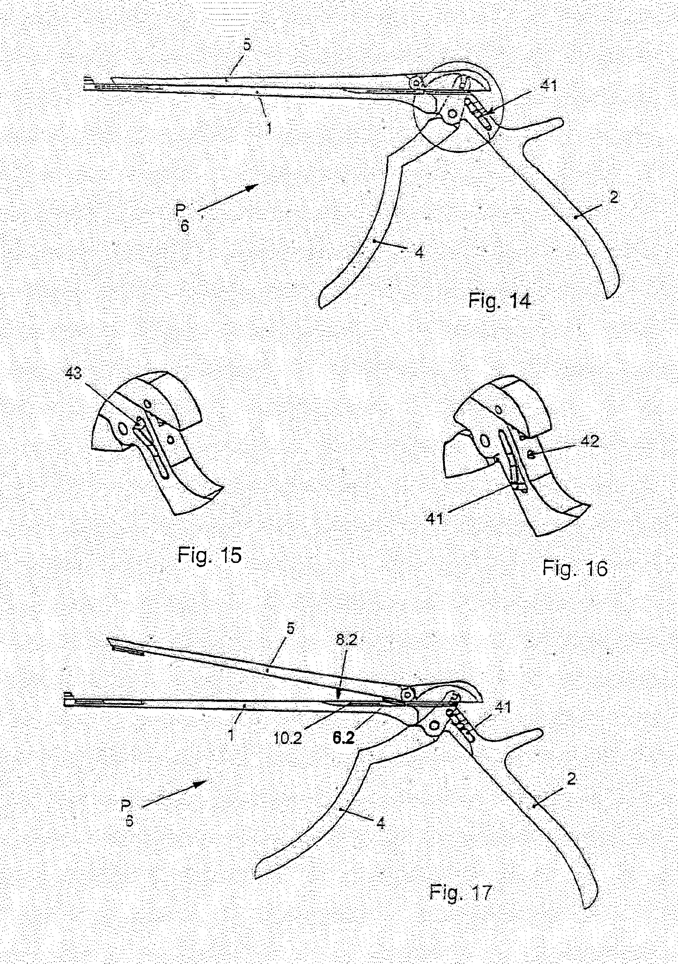

In einem weiteren sehr bevorzugten Ausführungsbeispiel eines chirurgischen Instruments P₆ gemäß den Figuren 14 bis 17 befindet sich in dem feststehenden Griffteil 2 ein Kipphebel 41, der als Anschlag für das bewegbare Griffteil 4 wirken kann. Dieser Kipphebel 41 kann um eine Achse 42 drehen, so dass seine obere Kante 43 entweder das bewegbare Griffteil 4 hintergreift oder es freigibt. Dabei sind drei Stellungen möglich. Gemäß Figur 16 befindet sich die Kante 43 versenkt in dem feststehenden Griffteil 2 und hintergreift dabei, wie in Figur 14 gezeigt, den oberen Teil des bewegbaren Griffteils 4. In diesem Augenblick kann das Führungselement 6.2 nicht aus den hinterschnittenen Nuten 10.2 herausgelangen, sondern ist in diesen Nuten 10.2 gefangen, so dass der Schieber 5 nicht von dem Schaft 1 getrennt werden kann.

Wird nun der Kipphebel 42 betätigt und nimmt eine Position an, wie sie in Figur 15 gezeigt ist, so wird der obere Teil des bewegbaren Griffteils 4, wie in Figur 17 angedeutet, freigegeben, so dass auch das Führungselement 6.2 in einen Bereich der Längsnut 8.2 gelangen kann, in dem es nicht mehr von den hinterschnittenen Nuten 10.2 gefangen ist.

Denkbar ist noch durch Abstufung der Kante 43 eine dritte Position, bei der das bewegbare Griffteil 4 noch weiter geöffnet werden kann, so dass das Führungselement 16 aus den hinterschnittenen Nuten 10.2 genommen werden kann und der Schieber 5 vollständig freigegeben wird.

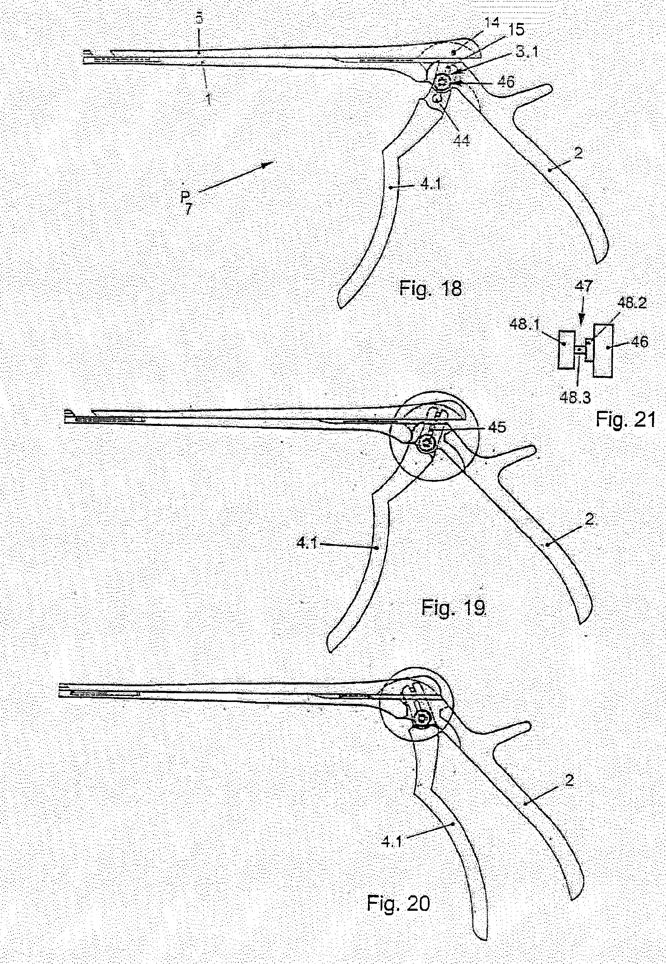

Ein nicht erfindungsgemäßes chirurgisches Instrument P₇ ist in den Figuren 18 bis 20 gezeigt. Dort ist das Drehgelenk 3.1 auf besondere Art und Weise ausgebildet, so dass dafür besonders Schutz begehrt wird. Hierzu ist in dem oberen Bereich des bewegbaren Griffteils 4.1 nicht nur eine Bohrung 44 eingeformt, welche einer nicht näher gekennzeichneten Bohrung in dem feststehenden Griffteil 2 entspricht, sondern an diese Bohrung 44 schließt sich ein Langloch 45 an. Dieser Ausgestaltung ist ein spezieller Druckknopf 46 zugeordnet, von dem ein in Figur 21 vergrößert dargestellter gestufter Stift 47 abragt.

Der Druckknopf 46 mit seinem Stift 47 steht unter dem Druck einer geeignet angeordneten Schraubenfeder. In Normallage befinden sich die Stiftteile 48.1 und 48.2 mit dem größeren Durchmesser in den Bohrungen 44 des bewegbaren Griffteils 4.1 des feststehenden Griffteils 2. Wird jedoch durch Bewegen dieser Anordnung das Stiftteil 48.3 mit dem geringeren Durchmesser, der etwa einer Breite des Langlochs 45 entspricht, in dessen Bereich gebracht, so kann das bewegbare Griffteil 4.1, wie in Figur 18 gezeigt, nach unten gezogen werden. Hierdurch rutscht der Querriegel 14 aus dem Schlitz 15 und der Schieber 5 wird freigegeben, so dass er von dem Schaft 1 abgenommen werden kann.

Bei diesem Ausführungsbeispiel ist auch in dem feststehenden Griffteil 2 eine Blattfeder 49 angeordnet, welche einer Drehbewegung des bewegbaren Griffteils 4 entgegenwirkt. Hierdurch erspart sich der Hersteller ein entsprechendes Spreizelement zwischen dem feststehenden Griffteil 2 und dem bewegbaren Griffteil 4.

**Bezugszeichen**

| | | | |
|---|---|---|---|
| 1 | Schaft | 26 | Steuerkante |
| 2 | Feststehendes Griffteil | 27 | Querbolzen |
| 3 | Drehgelenk | 28 | Anschlag |
| 4 | Bewegbares Griffteil | 29 | Zugknopf |
| 5 | Schieber | 30 | Stift |
| 6 | Führungselement | 31 | Ausnehmung |
| 7 | Unterfläche | 32 | Feder |
| 8 | Längsnut | 33 | Kipphebel |
| 9 | Schiene | 34 | Nase |
| 10 | Hinterschnittene Nut | 35 | Hebelarm |
| 11 | Teilbereich | 36 | Feder |
| 12 | Drehgelenk | 37 | Drehhebel |
| 13 | Schubteil | 38 | Drehachse |
| 14 | Querriegel | 39 | Druckraum |
| 15 | Schlitz | 40 | Raststift |
| 16 | Führungselement | 41 | Kipphebel |
| 17 | Verriegelungseinrichtung | 42 | Achse |
| | | 43 | Kante |
| 18 | Anschlag | 44 | Bohrung |
| 19 | Schieberknopf | 45 | Langloch |
| 20 | Raum | 46 | Druckknopf |
| 21 | Sackloch | 47 | Stift |
| 22 | Verbindungsstift | 48 | Stiftteile |
| 23 | Stanzmaul | 49 | Blattfeder |
| 24 | Zwischenraum | 50 | Druckknopf |
| 25 | Hintere Kante | P | Chirurg. Instrument |

## Patentansprüche

1. Chirurgisches Instrument mit einem Schaft (1, 1.1, 1.2) und einem an dem Schaft (1, 1.1, 1.2) durch eine Verriegelungseinrichtung (17, 17.1) begrenzbar gleitbar gelagerten Schieber (5, 5.1, 5.2), wobei zumindest ein in zumindest einer Längsnut (8.1, 8.2, 8.3) geführtes Führungselement (6.1, 6.2, 6.3) an Schaft (1, 1.1, 1.2) bzw. Schieber (5, 5.1, 5.2) vorgesehen ist und Schaft (1, 1.1, 1.2) bzw. Schieber (5, 5.1, 5.2) jeweils mit einem Griffteil (2, 4, 4.1) verbunden sind, von denen ein Griffteil (4, 4.1) gegenüber dem anderen Griffteil (2) bewegbar ist, wobei nach Aufhebung der Verriegelung (17, 17.1) der Schieber (5, 5.1, 5.2) und der Schaft (1, 1.1, 1.2) um ein Drehgelenk (12, 12.1) scherenartig voneinander trennbar sind, wobei über das Drehgelenk (12, 12.1) der Schieber (5, 5.1, 5.2) mit einem Schubteil (13) verbunden ist,
**dadurch gekennzeichnet, dass**
das Schubteil (13) einen Querriegel (14) aufweist, der in einen Schlitz (15) in dem bewegbaren Griffteil (4, 4.1) eingreift, und unterhalb des Querriegels (14) mit Führungselementen (16) versehen ist, die wiederum in hinterschnittenen Nuten (10.1) in Fortsetzung der Längsnut (8.2) geführt sind.

2. Chirurgisches Instrument nach Anspruch 1,
**dadurch gekennzeichnet, dass**
sich das Drehgelenk (12.1) zwischen dem Schaft (1.1) und einem feststehenden Griffteil (2) befindet.

3. Chirurgisches Instrument nach Anspruch 1,
**dadurch gekennzeichnet, dass**
sich das Drehgelenk (12) zwischen dem Schieber (5, 5.2) und einem bewegbaren Griffteil (4) befindet.

4. Chirurgisches Instrument nach Anspruch 3,
**dadurch gekennzeichnet, dass**
zwischen das Drehgelenk (12) und das bewegbare Griffteil (4) ein Schubteil (13) eingeschaltet ist.

5. Chirurgisches Instrument nach einem der vorhergehenden Ansprüche,
**dadurch gekennzeichnet, dass**
das Drehgelenk (12) aus einem Querbolzen (27) besteht, der gabelartig ineinander greifende Zungen miteinander verbindet.

6. Chirurgisches Instrument nach einem der vorhergehenden Ansprüche,
**dadurch gekennzeichnet, dass**
in bzw. aus der lichten Weite zumindest eines Teils der Längsnut (8.2, 8.3) ein Anschlag (18, 30, 50, 34, 37) hinter oder vor dem Führungselement (16, 6.3) bewegbar ist.

7. Chirurgisches Instrument nach Anspruch 6,
**dadurch gekennzeichnet, dass**
der Anschlag (18) in einem Sackloch (21) in dem Schaft (1) oder Schieber (5) geführt ist.

8. Chirurgisches Instrument nach Anspruch 6 oder 7,
**dadurch gekennzeichnet, dass**
der Anschlag (18) schräg zu der Längsnut (8.2) geführt ist.

9. Chirurgisches Instrument nach einem der Ansprüche 6 bis 8,
**dadurch gekennzeichnet, dass**
sich der Anschlag (18) gegen einen Kraftspeicher abstützt.

10. Chirurgisches Instrument nach Anspruch 9,
**dadurch gekennzeichnet, dass**
der Kraftspeicher eine Schraubenfeder ist.

11. Chirurgisches Instrument nach wenigstens einem der Ansprüche 6 bis 10.
**dadurch gekennzeichnet, dass**
der Anschlag (18) über einen Verbindungsstift (22) mit einem Schieberknopf (19) verbunden ist, der sich außen am Schaft (1) befindet.

12. Chirurgisches Instrument nach Anspruch 11,
**dadurch gekennzeichnet, dass**
der Verbindungsstift (22) einen Gewindeabschnitt aufweist, mit dem er in eine Gewindebohrung in dem Anschlag (18) einsetzbar ist.

13. Chirurgisches Instrument nach wenigstens einem der Ansprüche 6 bis 10,
**dadurch gekennzeichnet, dass**
der Anschlag (30) mit einem Zugknopf (29) außen an dem Schieber (5.2) in Verbindung steht.

14. Chirurgisches Instrument nach wenigstens einem der Ansprüche 6 bis 10,
**dadurch gekennzeichnet, dass**
in den Anschlag (50) eine Ausnehmung (31) eingeformt ist, welche bei einem Verschieben des Anschlags (50) eine Verlängerung der Längsnut (8.3) darstellt.

## Claims

1. Surgical instrument with a shaft (1, 1.1, 1.2) and a slide (5, 5.1, 5.2) that is supported such that it is limitably slideable at the shaft (1, 1.1, 1.2) by means of a locking device (17, 17.1), wherein at least one guiding element (6.1, 6.2, 6.3), which is guided in at least one longitudinal groove (8.1, 8.1, 8.3), is provided at the shaft (1, 1.1, 1.2) and/or the slide (5, 5.1, 5.2), the shaft (1, 1.1, 1.2) and/or the slide (5, 5.1, 5.2) each being connected to a respective handle part (2, 4, 4.1), of which handle parts (2, 4, 4.1) one handle part (4, 4.1) is moveable with respect to the other handle part (2), wherein after release of the locking (17, 17.1) the slide (5, 5.1, 5.2) and the shaft (1, 1.1, 1.2) are separable from each other about a rotational joint (12, 12.1) in a scissor-like manner, wherein the slide (5, 5.1, 5.2) is connected to a push part (13) via the rotational joint (12, 12.1),
**characterized in that** the push part (13) comprises a transversal lock bar (14) engaging in a slot (15) in the moveable handle part (4, 4.1), and is provided with guiding elements (16) below the transversal lock bar (14), which guiding elements (16) are guided in undercut grooves (10.1) in continuation of the longitudinal groove (8.2).

2. Surgical instrument according to claim 1,
**characterized in that**
the rotational joint (12.1) is situated between the shaft (1.1) and a stationary handle part (2).

3. Surgical instrument according to claim 1,
**characterized in that**
the rotational joint (12) is situated between the slide (5, 5.2) and a moveable handle part (4).

4. Surgical instrument according to claim 3,
**characterized in that**
the push part (13) is connected between the rotational joint (12) and the moveable handle part (4).

5. Surgical instrument according to one of the preceding claims,
**characterized in that**
the rotational joint (12) is implemented as a transversal bolt (27) joining tongues to each other that engage with each other in a fork-like manner.

6. Surgical instrument according to one of the preceding claims,
**characterized in that**
a stop (18, 30, 50, 34, 37) is moveable, behind or in front of the guiding element (16, 6.3), into respectively out of the inner width of at least a portion of the longitudinal groove (8.2, 8.3).

7. Surgical instrument according to claim 6,
**characterized in that**
the stop (18) is guided in a blind hole (21) in the shaft (1) or in the slide (5).

8. Surgical instrument according to claim 6 or 7,
**characterized in that**
the stop (18) is guided inclined with respect to the longitudinal groove (8.2).

9. Surgical instrument according to one of claims 6 to 8,
**characterized in that**
the stop (18) is braced against an energy accumulator.

10. Surgical instrument according to claim 9,
**characterized in that**
the energy accumulator is a helical spring.

11. Surgical instrument according to at least one of claims 6 to 10,
**characterized in that**
the stop (18) is connected to a slide button (19) by way of a connecting pin (22), which slide button (19) is situated outside at the shaft (1).

12. Surgical instrument according to claim 11,
**characterized in that**
the connecting pin (22) comprises a threaded section by which it is insertable into a threaded bore in the stop (18).

13. Surgical instrument according to at least one of claims 6 to 10,
**characterized in that**
the stop (30) is in connection with a pull button (29) outside at the slide (5.2).

14. Surgical instrument according to at least one of claims 6 to 10,
**characterized in that**
a recess (31) is moulded into the stop (50), which recess (31), in a case of a shifting of the stop (50), is an extension of the longitudinal groove (8.3).

## Revendications

1. Instrument chirurgical avec une hampe (1, 1.1, 1.2) et un coulisseau (5, 5.1, 5.2) supporté à la hampe (1, 1.1, 1.2) par un dispositif de verrouillage (17, 17.1) dans une manière limitablement glissable,
au moins un élément de guidage (6.1, 6.2, 6.3) guidé dans au moins une rainure longitudinale (8.1, 8.2, 8.3) étant prevu à la hampe (1, 1.1, 1.2) et/ou au coulisseau (5, 5.1, 5.2), et la hampe (1, 1.1, 1.2) et/ou le coulisseau (5, 5.1, 5.2) étant respectivement joint avec une pièce manche (2, 4, 4.1), une pièce manche (4, 4.1) desdits pièces manche (2, 4, 4.1) étant mobile relatif à l'autre pièce manche (2),
le coulisseau (5, 5.1, 5.2) et la hampe (1, 1.1, 1.2) étant séparable l'un de l'autre autour d'un joint rotatif (12, 12.1) dans une manière de ciseaux après désactivation du verrouillage (17, 17.1),
le coulisseau (5, 5.1, 5.2) étant joint avec une pièce de poussée (13) par le biais du joint rotatif (12, 12.1), **caractérisé en ce que**
la pièce de poussée (13) présente un verrou transversal (14) engageant dans une fente (15) dans la pièce manche (4, 4.1) mobile, et étant prévue avec des éléments de guidage (16) au-dessous du verrou transversal (14), lesquels éléments de guidage (16) sont guidés dans des rainures contre-dépouillées (10.1) en continuance de la rainure longitudinale (8.2)

2. Instrument chirurgical selon la revendication 1,
**caractérisé en ce que**
le joint rotatif (12.1) est situé entre la hampe (1.1) et une pièce manche (2) stationnaire.

3. Instrument chirurgical selon la revendication 1,
**caractérisé en ce que**
le joint rotatif (12) est situé entre le coulisseau (5, 5.2) et une pièce manche (4) mobile.

4. Instrument chirurgical selon la revendication 3,
**caractérisé en ce que**
la pièce de poussée (13) est connectée entre le joint rotatif (12) et la pièce manche (4) mobile.

5. Instrument chirurgical selon l'une quelconque des revendications précédentes,
**caractérisé en ce que**
le joint rotatif (12) consiste en un boulon transversal (27) joignant des languettes engagées entre eux dans une manière de fourchette.

6. Instrument chirurgical selon l'une quelconque des revendications précédentes,
**caractérisé en ce qu'**
une butée (18, 30, 50, 34, 37) peut être mouvée derriére ou avant l'élément de guidage (16, 16.3) dans ou hors de la largeur libre d'au moins une partie de la rainure longitudinale (8.2, 8.3).

7. Instrument chirurgical selon la revendication 6,
**caractérisé en ce que**
la butée (18) est guidée dans un trou bourgne (21) dans la hampe (1) ou dans le coulisseau (5).

8. Instrument chirurgical selon l'une quelconque des revendications 6 ou 7,
**caractérisé en ce que**
la butée (18) est guidée obliquement relatif à la rainure longitudinale (8.2).

9. Instrument chirurgical selon une des revendications 6 à 8,
**caractérisé en ce que**
la butée (18) s'appuie contre un mécanisme à ressort.

10. Instrument chirurgical selon la revendication 9,
**caractérisé en ce que**
le mécanisme à ressort est un ressort à boudin.

11. Instrument chirurgical selon au moins une des revendications 6 à 10,
**caractérisé en ce que**
la butée (18) est jointe, par le biais d'une broche joignant (22), avec un bouton coulisseau (19) qui est situé au-dehors à la hampe (1).

12. Instrument chirurgical selon la revendication 11,
**caractérisé en ce que**
la broche joignant (22) présente une section fileté, avec laquelle elle est insertable dans un trou taraudé dans la butée (18).

13. Instrument chirurgical selon au moins une des revendications 6 à 10,
**caractérisé en ce que**
la butée (30) est en connexion avec un bouton de traction (29) au-dehors au coulisseau (5.2).

14. Instrument chirurgical selon au moins une des revendications 6 à 10,
**caractérisé en ce que**
dans la butée (50) une échancrure (31) est moulée, laquelle en cas d'un déplacement de la butée (50) implémente un rallongement de la rainure longitudinale (8.3).
